# EUROPEAN PATENT SPECIFICATION

(11) **EP 3 006 619 B1**
(45) Date of publication and mention of the grant of the patent: **29.08.2018**
(21) Application number: 14425124.6
(22) Date of filing: 06.10.2014
(51) Int. Cl.: D06F 58/24, D06F 58/28

(54) **DEVICE AND METHOD FOR THE CONDENSATION OF STEAM IN THE DISCHARGE CIRCUIT OF A WASHING AND DISINFECTING MACHINE**
VORRICHTUNG UND VERFAHREN ZUR KONDENSATION VON DAMPF IM ENTLADEKREIS EINER WASCH- UND DESINFEKTIONSMASCHINE
DISPOSITIF ET PROCÉDÉ POUR LA CONDENSATION DE VAPEUR DANS LE CIRCUIT DE DÉCHARGE D'UNE MACHINE DE LAVAGE ET DE DÉSINFECTION

(43) Date of publication of application: 13.04.2016
(73) Proprietor: Bonferraro S.p.A., 37060 Bonferraro (VR) (IT)
(72) Inventor: Gobbi, Ezio, 46037 Roncoferraro MN (IT); Artoni, Michele, 42016 Guastalla RE (IT); Tosi, Mauro, 37051 Bovolone VR (IT)
(74) Representative: Concone, Emanuele

(56) References cited:
- DE-A1- 4 212 965
- DE-A1-102007 060 853
- US-A- 3 132 005
- US-A1- 2009 038 661

## Description

The present invention relates to washing and disinfecting machines, typically for items used in the medical field, and in particular to a device and method for the condensation of the steam present in the air that is discharged by said machines.

It is known that a washing and disinfecting machine operates at high temperatures of the wash/rinse water that imply the generation of a significant amount of steam, therefore in order to be installed in a room without fumes evacuation systems said machine must include also a steam condenser located in the air discharge circuit. In fact the presence of a device that provides the condensation of the steam prior to discharging it in the environment prevents the formation of condensate on the discharge outlet or in the adjacent regions, which may have adverse effects also on the operator and/or on other machines present in the room also due to the significant change in temperature and humidity.

This type of condenser generally operates with cold water that is atomized in the discharge circuit through which the steam passes, so that the cooling of the steam and of the discharge duct walls causes the condensation of the steam with subsequent discharge of the condensate and of the condensation water through a drain pipe. A water condenser therefore essentially consists of a condensation chamber with relevant inlet/outlet ducts, a water load pipe provided with a valve, a plurality of spraying nozzles fed through said pipe and arranged in said chamber, a pipe for draining water from the condensation chamber and a level sensor arranged in the chamber to detect the lack of water supply or drain.

A conventional condenser has a very simple operation, controlled by a relevant control unit, which consists in opening the water load valve for a preset time during two phases of the operating cycle of the machine in which it is installed:
a) the phase of heating the wash/rinse water, during which the steam that is generated due to the high temperature in the wash tank has to be discharged upon reaching a certain volume in order to prevent an excessive pressure in the wash tank, said intervention value being set through experimental tests; and
b) the drying phase, during which dry air is introduced in the tank while moist air is discharged, in particular during the first minutes of activation of the fan of the drying system.

This known arrangement has the drawback that in certain conditions an insufficient steam condensation or a water consumption greater than needed may occur, since the condenser is designed to operate with given nominal conditions and cannot take into account the actual operating conditions of the machine.

For example, an insufficient condensation may be due to one or more causes such as an amount of generated steam greater than expected, a condensation water temperature higher than expected or a decrease in the flow rate of the water sprayed by the nozzles due to a low pressure in the water mains system and/or a clogging of the water supply circuit (e.g. in the filters or the sprayers themselves). Vice versa, a portion of the water can be wasted if the amount of generated steam is lower than expected, the condensation water temperature is lower than expected or an increased pressure in the water mains system results in an increased flow rate.

A dishwasher with the features of the preamble of claim 1 is known from US2009/038661 A.

Therefore the object of the present invention is to provide a device and method for the condensation of steam that is free from said drawbacks. Said object is achieved by means of a device comprising a temperature probe arranged in the air discharge circuit downstream from the spraying nozzles, said probe being operatively connected to the control unit so as to implement the relevant operating method taking into account the detected temperature. Other advantageous features of the present device are specified in the dependent claims, in particular the use of a thermo-hygrometric probe that is able to detect not only the temperature but also the humidity of the air that is being discharged.

The main advantage of the present device is that of minimizing the water consumption and achieving an optimal steam condensation regardless of the operating conditions of the machine, thanks to the feedback provided by the probe that detects the characteristics of the air leaving the machine.

A second significant advantage of this device is that of having a simple structure that is easily applicable to any washing and disinfecting machine. This device can also be applied as an upgrade on an existing machine without requiring particular changes to the structure of the machine and/or to the arrangement of the components thereof

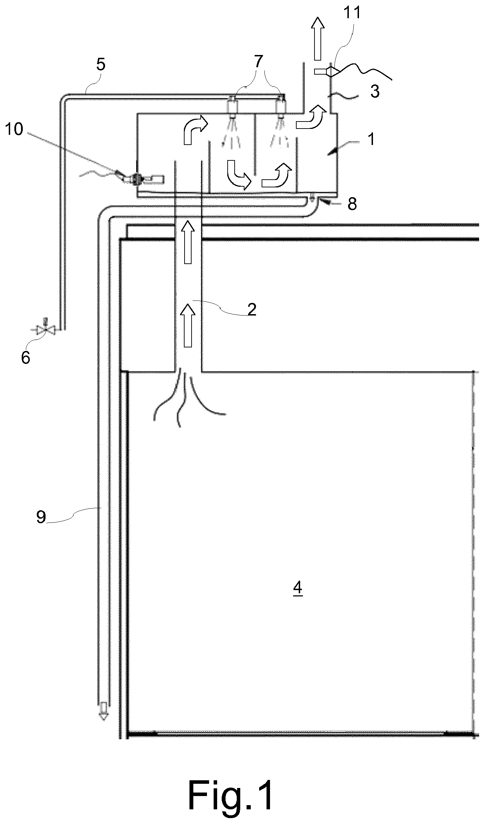

Further advantages and characteristics of the device and method according to the present invention will become apparent to those skilled in the art from the following detailed description of an embodiment thereof with reference to the only drawing, annexed as Fig.1, that shows a diagrammatic view of the device applied to a washing and disinfecting machine.

Referring to said figure, there is seen that a condensing device according to the invention conventionally includes a condensation chamber 1 with relevant inlet 2 and outlet 3 ducts that are part of the discharge circuit of the moist air (indicated by the arrows) coming from the wash tank 4 of a washing and disinfecting machine. A water pipe 5 connected to the water mains system and provided with a valve 6 feeds a plurality of spraying nozzles 7 (two in the illustrated example) arranged in chamber 1. The water sprayed by nozzles 7 and the condensed steam are discharged from chamber 1 through a drain 8 connected to a drain pipe 9, the water level in chamber 1 being monitored by a level sensor 10 operatively connected to a control unit (not shown) that controls also the opening and closing of valve 6.

The novel aspect of the present device, as mentioned above, lies in the presence of a temperature probe 11 arranged in the air discharge circuit downstream from the spraying nozzles 7, typically in the outlet duct 3. Said probe 11 is operatively connected to the control unit so as to provide a feedback on the condensation action carried out in chamber 1.

In practice, probe 11 detects the temperature of the air leaving the machine and transmits it to the control unit which can thus control the activation of nozzles 7 taking into account the fact that a temperature higher than expected indicates a decreased condensing effectiveness, due to one or more of the causes explained above, therefore it is necessary to extend the opening time of valve 6.

Vice versa, if the temperature of the outgoing air is lower than expected, in relation with the water temperature in the wash tank, it is possible to interrupt or at least reduce the flow rate towards nozzles 7 in order to decrease the water consumption (to this purpose, valve 6 is of an adjustable type). It is clear that the comparison between the detected temperature and the expected temperature, that takes place in the control unit, will cause an intervention on valve 6 only if the difference between said temperatures is greater than a minimum preset threshold.

This temperature check on the outgoing air is further preferably applied also in the water temperature maintenance phase, for example between a wash cycle and the following one, when the release of steam is in relation with various factors such as the outside temperature, the amount of water to be heated, the required thermal gradient, etc. In this case, the device according to the present invention allows to achieve an effective steam condensation also during a phase that is not taken into consideration in conventional devices.

The temperature probe 11 may obviously be of any type suitable for the purpose, such as a negative temperature coefficient (NTC) probe or a platinum resistance (Pt) probe. Moreover, in order to further optimize the control and effectiveness of the device, probe 11 may be a thermo-hygrometric probe that detects not only the temperature but also the humidity of the outgoing air, or a separate hygrometer probe can be combined with the temperature probe 11.

The operating method of a device according to the present invention may therefore be summarized in the following steps:
a) opening the load valve 6 of the condensation water at a preset moment of the wash/rinse water heating phase or of the drying phase of the operating cycle of a washing and disinfecting machine;
b) detecting the air temperature downstream from the condensation nozzles 7 and transmitting the detected value to the control unit;
c) comparing in the control unit the detected temperature with the expected temperature, so as to check whether the difference between said temperatures is greater than a minimum preset threshold;
d) in case the difference is positive and greater than the threshold mentioned in step c), extending the opening time of valve 6 until said difference drops below the threshold; or
d') in case the difference is negative and greater than the threshold mentioned in step c), shortening the opening time of valve 6 and/or decreasing the flow rate of the condensation water;
e) closing the load valve 6 of the condensation water at the end of a preset opening time or at the end of said time as modified in step d) or d').

As mentioned above, this operating method is preferably applied also in a water temperature maintenance phase different from the phases mentioned in step a). Furthermore, it may include also steps of detecting and comparing the humidity of the outgoing air, with a possible corresponding intervention on the load valve 6, if the device includes also a hygrometer probe (integrated or not with the temperature probe).

It is clear that the embodiment of the device according to the invention described and illustrated above is just an example susceptible of various modifications. In particular, the shape, size and arrangement of the condensation chamber 1 and of the relevant ducts/pipes 2, 3, 5, and 9 can be varied according to the needs, as well as the type and exact position of probe 11 (and of the possible hygrometer probe) as long as it is arranged downstream from nozzles 7.

## Claims

1. Steam condensation device comprising a condensation chamber (1) with relevant inlet (2) and outlet (3) ducts that are part of a discharge circuit of moist air coming from a wash tank (4) of a washing and disinfecting machine, a drain (8) formed in the bottom of the condensation chamber (1) and connected to a drain pipe (9), a plurality of spraying nozzles (7) arranged in said condensation chamber (1) and **characterised in that** the nozzles are fed through a water pipe (5) connected to the water mains system and provided with a valve (6), and further comprising a level sensor (10) suitable to detect the water level in the condensation chamber (1), as well as a control unit operatively connected to said valve (6) and to said level sensor (10), and further including at least one temperature probe (11) arranged downstream from said spraying nozzles (7) along said moist air discharge circuit, preferably in said outlet duct (3), said temperature probe (11) being operatively connected to said control unit.

2. Device according to claim 1, **characterized in that** it further includes a hygrometer probe arranged downstream from the spraying nozzles (7) along the moist air discharge circuit, preferably in the outlet duct (3).

3. Device according to claim 2, **characterized in that** the hygrometer probe and the temperature probe (11) are integrated into a single probe.

4. Device according to any of the preceding claims, **characterized in that** the valve (6) is an adjustable valve.

5. Operating method of a device according to any of the preceding claims, **characterized in that** it includes the following steps:
a) opening the load valve (6) of the condensation water at a preset moment of the wash/rinse water heating phase or of the drying phase of the operating cycle of a washing and disinfecting machine;
b) detecting the air temperature downstream from the condensation nozzles (7) and transmitting the detected value to the control unit;
c) comparing in the control unit the detected temperature with the expected temperature, so as to check whether the difference between said temperatures is greater than a minimum preset threshold;
d) in case the difference is positive and greater than the threshold mentioned in step c), extending the opening time of the valve (6) until said difference drops below the threshold; or
d') in case the difference is negative and greater than the threshold mentioned in step c), shortening the opening time of the valve (6) and/or decreasing the flow rate of the condensation water;
e) closing the load valve (6) of the condensation water at the end of a preset opening time or at the end of said time as modified in step d) or d').

6. Method according to claim 5, **characterized in that** it is carried out also in a water temperature maintenance phase that is different from the phases mentioned in step a).

7. Method according to claim 5 or 6 of operation of a device according to claim 2 or 3, **characterized in that** it further includes steps of detecting and comparing the humidity of the outgoing air, with a possible corresponding intervention on the load valve (6).

8. Washing and disinfecting machine, **characterized in that** it includes a steam condensation device having a structure according to any of claims 1 to 4 and an operating method according to any of claims 5 to 7.

## Patentansprüche

1. Dampf-Kondensations-Vorrichtung, umfasssend eine Kondensationskammer (1) mit entsprechenden Einlass- (2) und Auslasskanälen (3), welche Teil eines Ausströmkreises für feuchte Luft sind, die aus dem Waschbehälter (4) einer Wasch- und Desinfekionsmaschine kommt, einen Ablauf (8), der in dem Boden der Kondensationskammer (1) ausgebildet und an eine Ablaufleitung (9) angeschlossen ist, eine Mehrzahl von Sprühdüsen (7), die in der besagten Kondensationskammer (1) angeordnet sind, und **dadurch gekennzeichnet, dass** die Düsen durch eine Wasserleitung (5) gespeist werden, die an dem Wasserleitungsnetz angeschlossen ist und mit einem Ventil (6) versehen ist, und ferner umfassend einen Pegelsensor (10), der dafür geeignet ist, den Wasserpegel in der Kondensationskammer (1) zu ermitteln, sowie eine wirkungstechnisch an dem besagten Ventil (6) und dem besagten Pegelsensor (10) angeschlossene Steuereinheit, sowie ferner umfassend wenigstens einen Temperaturfühler (11), der entlang des besagten Ausströmkreises für feuchte Luft stromabwärts der besagten Sprühdüsen (7), bevorzugt in dem Auslasskanal (3), angeordnet ist, wobei der besagte Temperaturfühler (11) an die besagte Steuereinheit wirkungstechnisch angeschlossen ist.

2. Vorrichtung gemäß Anspruch 1, **dadurch gekennzeichnet, dass** sie ferner einen Feuchtigkeitsmessfühler aufweist, der entlang des Ausströmkreises für feuchte Luft stromabwärts der Sprühdüsen (7), bevorzugt in dem Auslasskanal (3), angeordnet ist.

3. Vorrichtung gemäß Anspruch 2, **dadurch gekennzeichnet, dass** der Feuchtigkeitsmessfühler und der Temperaturfühler (11) zu einem einzigen Fühler integriert sind.

4. Vorrichtung gemäß einem der vorhergehenden Ansprüche, **dadurch gekennzeichnet, dass** das Ventil (6) ein einstellbares Ventil ist.

5. Betriebsverfahren für eine Vorrichtung nach einem der vorhergehenden Ansprüche, **dadurch gekennzeichnet, dass** es die folgenden Schritte umfasst:
a) Öffnen des Beschickungsventils (6) des Kondensationswassers zu einem vorgegebenen Zeitpunkt der Aufheizphase des Wasch-/Spülwassers oder der Trocknungsphase des Arbeitszyklus der Wasch- und Desinfektionsmaschine;
b) Ermitteln der Lufttemperatur stromabwärts der Kondensationsdüsen (7) und Übermitteln des ermittelten Wertes an die Steuereinheit;
c) Vergleichen der ermittelten Temperatur mit der erwarteten Temperatur in der Steuereinheit, um zu überprüfen, ob die Differenz zwischen den Temperaturen größer ist als ein minimaler, vorgegebener Schwellwert;
d) falls die Differenz positiv ist und größer als der im Schritt c) erwähnte Schwellwert, Verlängern der Öffnungszeit des Ventils (6) bis die besagte Differenz unter den Schwellwert fällt; oder
d') falls die Differenz negativ ist und größer als der im Schritt c) erwähnte Schwellwert, Verkürzen der Öffnungszeiten des Ventils (6) und/oder Senken der Durchflussrate des Kondensationswassers;
e) Schließen des Beschickungsventils (6) der Kondensationswassers am Ende der voreingestellten Öffnungszeit oder am Ende der besagten Zeit, wie sie im Schritt d) oder d') modifiziert wurde.

6. Verfahren gemäß Anspruch 5, **dadurch gekennzeichnet, dass** es auch in einer Wassertemperatur-Erhaltungsphase ausgeführt wird, die sich von den in dem Schritt a) erwähnten Phasen unterscheidet.

7. Verfahren gemäß Anspruch 5 oder 6 zum Betreiben dder Vorrichtung nach Anspruch 2 oder 3, **dadurch gekennzeichnet, dass** es ferner Schritte des Ermittelns und Vergleichens der Feuchtigkeit der ausströmenden Luft aufweist, mit einen möglichen, entsprechenden Eingriff an dem Beschickungsventil (6).

8. Wasch- und Desinfektionsmaschine, **dadurch gekennzeichnet, dass** sie eine Dampf-Kondensations-Vorrichtung aufweist mit einer Struktur gemäß einem der Ansprüche 1 bis 4 und mit einer Betriebsweise gemäß einem der Ansprüche 5 bis 7.

## Revendications

1. Dispositif de condensation de vapeur comprenant une chambre de condensation (1) avec des conduits d'entrée (2) et de sortie (3) correspondants qui font partie d'un circuit de décharge d'air humide provenant d'un réservoir de lavage (4) d'une machine de lavage et de désinfection, un drain (8) formé au fond de la chambre de condensation (1) et raccordé à un tuyau de drain (9), une pluralité de buses de pulvérisation (7) agencées dans ladite chambre de condensation (1) et **caractérisé en ce que** les buses sont alimentées par un tuyau d'eau (5) raccordé au système d'alimentation en eau et prévues avec une valve (6), et comprenant en outre :
un capteur de niveau (10) approprié pour détecter le niveau d'eau dans la chambre de condensation (1), ainsi qu'une unité de commande raccordée, de manière opérationnelle, à ladite valve (6) et audit capteur de niveau (10), et comprenant en outre :
au moins une sonde de température (11) agencée en aval desdites buses de pulvérisation (7) le long dudit circuit de décharge d'air humide, de préférence dans ledit conduit de sortie (3), ladite sonde de température (11) étant raccordée, de manière opérationnelle, à ladite unité de commande.

2. Dispositif selon la revendication 1, **caractérisé en ce qu'**il comprend en outre une sonde d'hygrométrie agencée en aval des buses de pulvérisation (7) le long du circuit de décharge d'air humide, de préférence dans le conduit de sortie (3).

3. Dispositif selon la revendication 2, **caractérisé en ce que** la sonde d'hygrométrie et la sonde de température (11) sont réalisées d'un seul tenant en une seule sonde.

4. Dispositif selon l'une quelconque des revendications précédentes, **caractérisé en ce que** la valve (6) est une valve ajustable.

5. Procédé de fonctionnement d'un dispositif selon l'une quelconque des revendications précédentes, **caractérisé en ce qu'**il comprend les étapes suivantes :
a) ouvrir la valve de charge (6) de l'eau de condensation à un moment prédéterminé de la phase de chauffage de l'eau de lavage / rinçage ou de la phase de séchage du cycle de fonctionnement d'une machine de lavage et de désinfection ;
b) détecter la température d'air en aval des buses de condensation (7) et transmettre la valeur détectée à l'unité de commande ;
c) comparer, dans l'unité de commande, la température détectée avec la température attendue, afin de vérifier si la différence entre lesdites températures est supérieure à un seuil minimum prédéterminé ;
d) dans le cas dans lequel la différence est positive et supérieure au seuil mentionné à l'étape c), prolonger le temps d'ouverture de la valve (6) jusqu'à ce que ladite différence chute au-dessous du seuil ; ou bien
d') dans le cas dans lequel la différence est négative et supérieure au seuil mentionné à l'étape c), raccourcir le temps d'ouverture de la valve (6) et/ou diminuer le débit de l'eau de condensation ;
e) fermer la valve de charge (6) de l'eau de condensation à la fin d'un temps d'ouverture prédéterminé ou à la fin dudit temps, tel que modifié à l'étape d) ou d').

6. Procédé selon la revendication 5, **caractérisé en ce qu'**il est réalisé également dans une phase de maintien de température d'eau qui est différente des phases mentionnées à l'étape a).

7. Procédé selon la revendication 5 ou 6 pour actionner un dispositif selon la revendication 2 ou 3, **caractérisé en ce qu'**il comprend en outre des étapes pour détecter et comparer l'humidité de l'air sortant, avec une intervention correspondante possible sur la valve de charge (6).

8. Machine de lavage et de désinfection, **caractérisée en ce qu'**elle comprend un dispositif de condensation de vapeur ayant une structure selon l'une quelconque des revendications 1 à 4 et un procédé de fonctionnement selon l'une quelconque des revendications 5 à 7.
